# EUROPEAN PATENT SPECIFICATION

(11) **EP 1 911 399 B1**
(45) Date of publication and mention of the grant of the patent: **23.12.2015**
(21) Application number: 07254033.9
(22) Date of filing: 11.10.2007
(51) Int. Cl.: A61B 5/053, A61B 5/08, A61N 1/368

(54) **Techniques for correlating thoracic impedance with physiological status**
Verfahren zur Korrelation von Thoraximpedanz mit physiologischem Zustand
Techniques pour la corrélation de l'impédance thoracique avec l'état physiologique

(30) Priority: 11.10.2006 US 829128 P; 29.12.2006 US 618266
(43) Date of publication of application: 16.04.2008
(73) Proprietor: PACESETTER, INC., Sylmar, CA 91342-9221 (US)
(72) Inventor: Min, Xiaoyi, Thousand Oaks CA, 91362 (US); Levine, Paul A., Santa Clarita CA, 91321 (US); Ostrow, Eliot L., Sunnyvale CA, 94087 (US)
(74) Representative: Noble, Nicholas

(56) References cited:
- EP-A- 0 985 429
- WO-A-98/19737

## Description

The subject matter presented herein generally relates to correlating thoracic impedance with physiological status.

To an increasing degree, implantable medical devices (IMDs) are being used to treat congestive heart failure. In that these are very complex patients whose underlying disease may tend to worsen long before this becomes clinically manifest, it is important to develop ways of monitoring the progressive deterioration before it causes clinical symptoms in order to notify the patient and/or clinician so that an intervention can be implemented to halt progressive deterioration. This intervention may include alterations in the functional parameters of the device and or adjustment of medications. A marker of progressive clinical deterioration is an inability to the heart to effectively pump the blood in a forward direction to meet the needs of the body. This allows the blood to back up into the lungs causing increasing congestion manifested by progressive shortness of breath. With the increased water in the lungs, the thoracic impedance will decrease. While measurement of thoracic impedance has been used for years to identify the need for a rate change , IMDs used in the treatment of heart failure have begun to use changes in thoracic impedance data to diagnose increasing lung water that if not treated, would eventuate in pulmonary edema and symptomatic heart failure. For instance, pulmonary edema (e.g., increased fluid levels in the lungs) produces decreased thoracic impedance values for electrical signals passing through the pulmonary tissues. The current systems are relatively black and white; they simply measure thoracic impedance and interpret any change as indicative of heart failure. There are a number of other reasons that transthoracic impedance will change and while causing shortness of breath, may not be overt heart failure or unmask the early stages of heart failure. Thus, the patient's physiological status may also affect pulmonary fluid levels. Accordingly, correlating physiological status with thoracic impedance measurements would allow more accurate detection of pulmonary fluid levels.

Exemplary techniques for correlating thoracic impedance values with physiological status are described. One technique involves an implantable medical device (IMD) that includes means for correlating thoracic impedance values with a patient's physiological status and means for interpreting the correlated thoracic impedance values utilizing a patient-based threshold to detect a heart failure condition.

Another technique correlates patient posture and thoracic impedance values for individual days to determine a first average thoracic impedance value for a generally horizontal patient posture and a second average thoracic impedance value for a generally vertical patient posture. For individual days the technique also calculates a difference between the first average thoracic impedance value and the second average thoracic impedance value. The technique further tracks changes to the difference over a plurality of days to detect a pulmonary edema condition of the patient.

In general, the various techniques, methods, devices, systems, etc., described herein, and equivalents thereof, are optionally suitable for correlating thoracic impedance with physiological status.

The invention is defined in claim 1. Methods disclosed hereinafter do not form part of the scope of the invention, which is defined in the appended claims. A device accoring to the preamble of claim 1 is known from EP0985429.

The mechanism to detect may utilize a scaling factor that takes into account an incidence of pulmonary edema prior to implantation, and the scaling factor may comprise a weighting coefficient. Preferably, the weighting coefficient is based upon patient information including echo parameters obtained in proximity to implantation of the implantable medical device.

There is further disclosed an implantable medical device (IMD) comprising: a mechanism operative to determine when a patient is in a supine posture; a mechanism operative to measure thoracic impedance values when the patient is in the supine posture; and a mechanism operative to determine an average thoracic impedance value from the measured thoracic impedance values for a first day and an average thoracic impedance value for a second day and to compare the averages to detect heart failure in the patient.

The mechanism operative to determine preferably compares the averages in light of a patient-based threshold and detects when a difference between the averages exceeds the patient-based threshold. The mechanism operative to determine may exclude thoracic impedance values measured within a pre-defined period of time after the patient assumes the supine posture.

There is further disclosed an implantable medical device (IMD) comprising: means for correlating thoracic impedance values with a patient's physiological status; and, means for interpreting the correlated thoracic impedance values utilizing a patient-based threshold to detect a heart failure condition.

The means for correlating may be configured to correlate the thoracic impedance values with at least two different aspects of the patient's physiological status. Preferably, at least two different aspects comprise patient posture and activity level. Preferably, the patient's physiological status comprises patient posture. Preferably, the means for correlating is configured to receive posture data from at least two posture sensing mechanisms, or may be configured to determine patient posture based upon one or more of: sensed posture data and time of day. Preferably, the means for interpreting is operable to calculate a first daily average thoracic impedance value when the patient is in an upright posture and a second daily average thoracic impedance value when the patient is in a supine posture and to determine is a difference between the first and second averages exceeds the patient-based threshold.

Preferably, the IMD further comprises a means for determining a difference between a first daily average thoracic impedance value when the patient is in an upright posture and a second daily average thoracic impedance value when the patient is supine and a means for notification if a difference between the first and second averages exceeds the patient-based threshold. The IMD may further compromise means for detecting a first daily average thoracic impedance value when the patient is in a upright posture and a second daily average thoracic impedance value when the patient is supine and a means for determining a difference between the first and second averages.

The IMD may further compromise means for adjusting one or more parameters of the IMD in an instance where the difference between the first and second averages exceeds the patient-based threshold in an effort to improve cardiac function. Preferably, the one or more parameter comprises one of: pacing rate and pacing AV delays.

There may be further enabled a computer-implemented method comprising: sensing thoracic impedance values from a patient; determining the patients posture; correlating the patient's posture and the thoracic impedance values to calculate a first average thoracic impedance for a first posture and a second average impedance for a second posture; and, detecting a pulmonary edema condition of the patient by comparing the first average to the second average.

In this method the determining may comprise monitoring times when the patient normally sleeps and receiving signals from a posture sensor. The correlating may exclude thoracic impedance values that occur within a pre-defined period after a change in posture is detected, and may comprise determining a difference between the first average and the second average.

Sensing, determining and correlating may be repeated for a plurality of individual days effective to determine the difference for individual days and wherein the detecting a pulmonary edema condition may comprise comparing the difference for each of the plurality of individual days.

The sensing may comprise sensing thoracic impendence values with an implanted medical device (IMD) and detecting a pulmonary edema condition may further comprise considering a patient condition prior to implantation of the IMD. The considering may comprise weighting the averages with clinical parameters established prior to implantation of the IMD. The detecting may comprise generating a patient-based threshold based upon patient information and determining whether a difference between the first and second averages exceeds the patient-based threshold.

There is further disclosed a computer-readable media that when stored on an implantable medical device (IMD) causes the IMD to perform acts comprising: for individual days, correlating patient posture and thoracic impedance values to determine a first average thoracic impedance value for a generally horizontal patient posture and a second average thoracic impedance value for a generally vertical patient posture, and for individual days calculating a difference between the first average thoracic impedance value and the second average thoracic impedance value; and, tracking changes to the difference over a plurality of days to detect a pulmonary edema condition of the patient.

Preferably, the correlating excludes thoracic impedance values for a predetermined period of time after a posture change occurs. Preferably, the tracking changes comprises detecting instances when the first average thoracic impedance value decreases from day to day while the second average thoracic impedance value remains relatively constant from day to day. Preferably, the tracking changes comprises generating a patient-based threshold based upon information obtained from the patient prior to the individual days and tracking whether the difference exceeds the patient-based threshold.

There is further disclosed a method comprising: obtaining patient information prior to a sample period where thoracic impedance values are measured; generating an individualized patient-based threshold utilizing the patient information; and, interpreting the thoracic impedance values from the sample period in light of the individualized patient-based threshold.

The obtaining may comprise obtaining patient information during implantation of an implantable medical device (IMD), and wherein the thoracic impedance values are measure by the IMD, or obtaining patient information after implantation of an implantable medical device (IMD) and before the sample period. The method may comprise correlating the thoracic impedance values with patient postures.

There is further disclosed, in an implantable medical device, a method comprising: sensing thoracic impedance values from a patient; determining the patient's posture; correlating the patient's posture and the thoracic impedance values to calculate a first average thoracic impedance for a first posture and a second average impedance for a second posture; and, detecting a pulmonary edema condition of the patient by comparing the first average to the second average.

Preferably, the determining comprises monitoring times when the patient normally sleeps and receiving signals from a posture sensor. Preferably, the correlating excludes thoracic impedance values that occur within a pre-defined period after a change in posture is detected, and may comprise sensing thoracic impendence values with an implanted medical device (IMD) and wherein the detecting a pulmonary edema condition further comprises considering a patient condition prior to implantation of the IMD. Preferably, the detecting comprises generating a patient-based threshold based upon patient information and determining whether a difference between the first and second averages exceeds the patient-based threshold.

Features and advantages of the described implementations can be more readily understood by reference to the following description taken in conjunction with the accompanying drawings.
Fig. 1 is a simplified diagram illustrating an exemplary implantable IMD operable to correlate thoracic impedance with physiological status in accordance with one embodiment.
Fig. 2 is a functional block diagram of an exemplary implantable IMD illustrating basic elements that are operable to correlate thoracic impedance with physiological status in accordance with one embodiment.
Figs. 3-5 are plots of thoracic impedance over time that can be utilized to correlate thoracic impedance with physiological status in accordance with various embodiments.
Figs. 6-7 are exemplary methods for correlating thoracic impedance with physiological status in accordance with one embodiment.

The following description includes the best mode presently contemplated for practicing the described implementations. This description is not to be taken in a limiting sense, but rather is made merely for the purpose of describing the general principles of the implementations. The scope of the described implementations should be ascertained with reference to the issued claims. In the description that follows, like numerals or reference designators will be used to reference like parts or elements wherever feasible.

### Overview

Various exemplary techniques, methods, devices, systems, etc., described herein pertain to correlating thoracic impedance measurements with physiological status. Thoracic impedance measurements can be obtained by an implanted medical device (IMD). For instance, an electrical signal can be discharged from a first electrode of the IMD. A portion of the electrical signal travels through intervening patient tissue and can be detected by a second electrode of the IMD. Impedance values of the intervening patient tissue can be derived from the detected signal. For IMDs employed in a cardiac environment, the first and second electrodes can be selected so that lung tissue lies therebetween. The impedance of the signals that travel through the lung tissue is affected by the fluid content of that lung tissue. Compromised heart function (e.g., heart failure) can lead to increased fluid in the lungs. For example, as the left side of the heart falls behind in pumping blood to the body, fluid backs up in the pulmonary vein and diffuses into the lungs.

Various aspects of the patient's physiological status also affect the fluid level in the lungs. For example, gravity causes some pooling of fluids in the patient's lower extremities when the patient is upright, such as in standing and sitting postures. This will remove fluid from the lungs causing a rise in thoracic impedance. When the patient lies down, such as in a supine posture, these fluids are mobilized over a period of minutes. When the patient assumes a horizontal supine posture the patient may experiences some fluid build-up in the pulmonary circulation and hence decreasing impedance values for a period of time as increased fluid is mobilized from the periphery and collects in the lungs. In a patient with normal heart function, the fluid levels and impedance values soon return to normal levels as the heart compensates with various mechanisms. In patients with compromised heart function, the heart may be unable to compensate sufficiently and the fluid may remain in the lung tissue as evidenced by continuing lowered and/or decreasing impedance values. In either instance, (e.g., whether the patient has normal heart function or compromised heart function) the impedance measurements have more prognostic value when correlated with the patient posture and changes to the patient posture. Accordingly, knowing what posture a patient is in when an impedance value is recorded as well as how long the patient has been in that posture, along with the relative rate of change of the impedance value, are useful in deriving prognostic value from the impedance value.

To summarize, the described implementations can correlate patient thoracic impedance values with the patient's physiological status to provide a context for the impedance values. The context allows more meaningful interpretation of the impedance values in diagnosing cardiac function.

### Exemplary IMD

The techniques described below can be implemented in connection with any IMD that is configured or configurable to sense cardiac data and/or provide cardiac therapy.

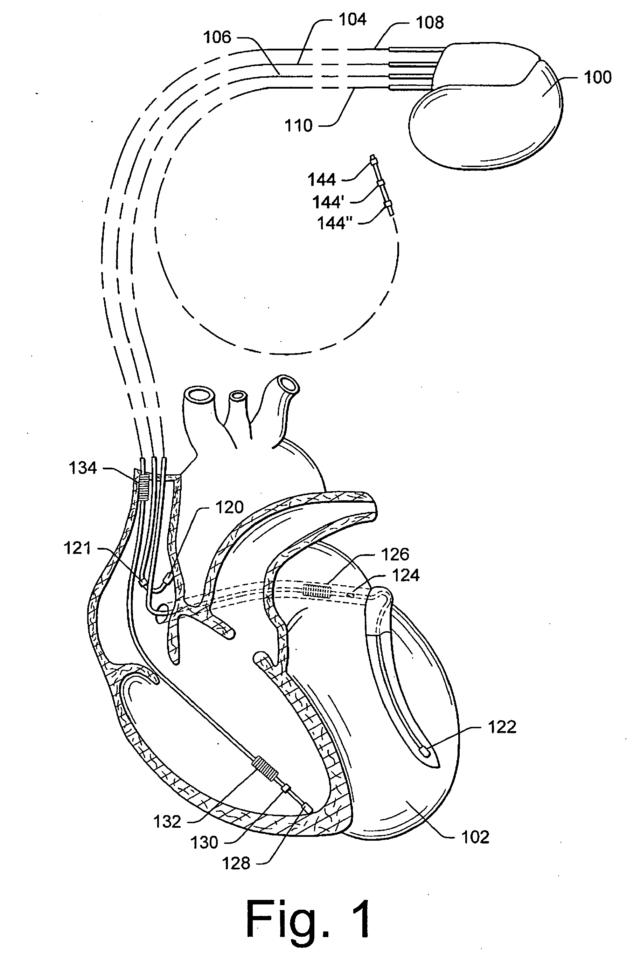

Fig. 1 shows an exemplary IMD 100 in electrical communication with a patient's heart 102 by way of three leads 104, 106, 108, suitable for delivering multi-chamber stimulation and shock therapy. The leads 104, 106, 108 are optionally configurable for delivery of stimulation pulses suitable for stimulation of autonomic nerves, non-myocardial tissue, other nerves, etc. In addition, IMD 100 includes a fourth lead 110 having, in this implementation, three electrodes 144, 144', 144" suitable for stimulation of autonomic nerves, non-myocardial tissue, other nerves, etc. For example, this lead may be positioned in and/or near a patient's heart or near an autonomic nerve within a patient's body and remote from the heart. In another example, the fourth lead can be configured to sense the phrenic nerve and/or activation of the diaphragm. The right atrial lead 104, as the name implies, is positioned in and/or passes through a patient's right atrium. The right atrial lead 104 optionally senses atrial cardiac signals and/or provides right atrial chamber stimulation therapy. As shown in Fig. 1, the IMD 100 is coupled to an implantable right atrial lead 104 having, for example, an atrial tip electrode 120, which typically is implanted in the patient's right atrial appendage. The lead 104, as shown in Fig. 1, also includes an atrial ring electrode 121. Of course, the lead 104 may have other electrodes as well. For example, the right atrial lead optionally includes a distal bifurcation having electrodes suitable for stimulation of autonomic nerves, non-myocardial tissue, other nerves, etc. In an alternative configuration, lead 110 can be replaced with a mechanism for connecting the IMD to various other devices. For example, the mechanism can facilitate connecting IMD 100 to a drug pump for dispensing drugs into the patient in accordance with instructions received from the IMD. The skilled artisan should recognize various other configurations that may be employed which are consistent with the principles described above and below.

To sense atrial cardiac signals, ventricular cardiac signals and/or to provide multi-site pacing therapy, particularly on the left side of a patient's heart, the IMD 100 is coupled to a coronary sinus lead 106 designed for placement in the coronary sinus and/or tributary veins of the coronary sinus. Thus, the coronary sinus lead 106 is optionally suitable for positioning at least one distal electrode adjacent to the left ventricle and/or additional electrode(s) adjacent to the left atrium. In a normal heart, tributary veins of the coronary sinus include, but may not be limited to, the great cardiac vein, the left marginal vein, the left posterior ventricular vein, the middle cardiac vein, and the small cardiac vein.

Accordingly, an exemplary coronary sinus lead 106 is optionally designed to receive atrial and ventricular cardiac signals and to deliver left ventricular pacing therapy using, for example, at least a left ventricular tip electrode 122, left atrial pacing therapy using at least a left atrial ring electrode 124, and shocking therapy using at least a left atrial coil electrode 126. The coronary sinus lead 106 further optionally includes electrodes for stimulation of autonomic nerves. Such a lead may include pacing and autonomic nerve stimulation functionality and may further include bifurcations or legs. For example, an exemplary coronary sinus lead includes pacing electrodes capable of delivering pacing pulses to a patient's left ventricle and at least one electrode capable of stimulating an autonomic nerve. An exemplary coronary sinus lead (or left ventricular lead or left atrial lead) may also include at least one electrode capable of stimulating an autonomic nerve, non-myocardial tissue, other nerves, etc., wherein such an electrode may be positioned on the lead or a bifurcation or leg of the lead.

IMD 100 is also shown in electrical communication with the patient's heart 102 by way of an implantable right ventricular lead 108 having, in this exemplary implementation, a right ventricular tip electrode 128, a right ventricular ring electrode 130, a right ventricular (RV) coil electrode 132, and an SVC coil electrode 134. Typically, the right ventricular lead 108 is transvenously inserted into the heart 102 to place the right ventricular tip electrode 128 in the right ventricular apex so that the RV coil electrode 132 will be positioned in the right ventricle and the SVC coil electrode 134 will be positioned in the superior vena cava. Accordingly, the right ventricular lead 108 is capable of sensing or receiving cardiac signals, and delivering stimulation in the form of pacing and shock therapy to the right ventricle. An exemplary right ventricular lead may also include at least one electrode capable of stimulating an autonomic nerve, non-myocardial tissue, other nerves, etc., wherein such an electrode may be positioned on the lead or a bifurcation or leg of the lead.

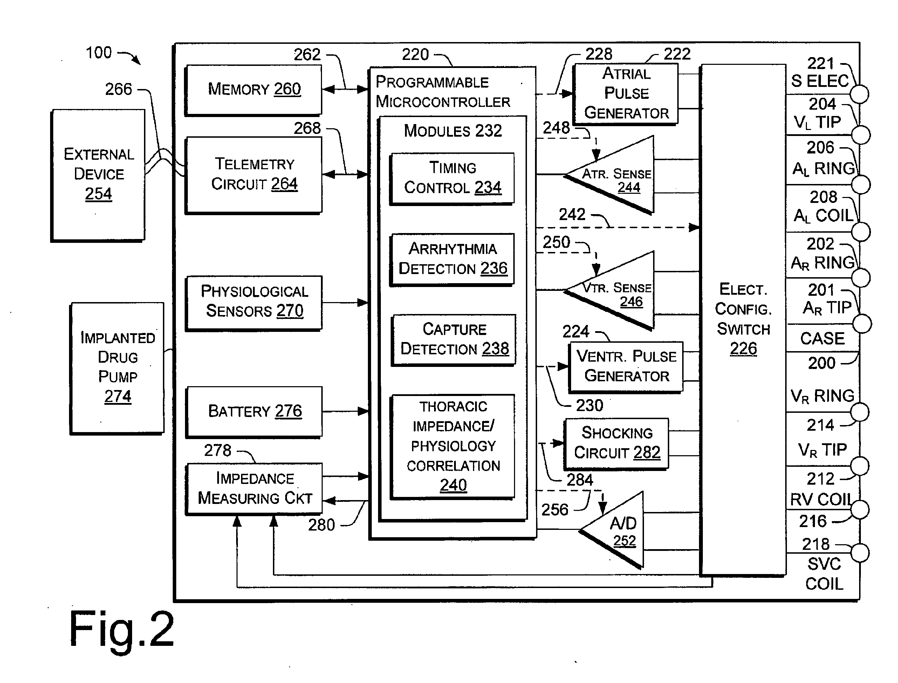

Fig. 2 shows an exemplary, simplified block diagram depicting various components of IMD 100. The IMD 100 can be capable of treating both fast and slow arrhythmias with stimulation therapy, including cardioversion, defibrillation, and pacing stimulation. The IMD can be solely or further capable of delivering stimuli to autonomic nerves, non-myocardial tissue, other nerves, etc. While a particular multi-chamber device is shown, it is to be appreciated and understood that this is done for illustration purposes only. Thus, the techniques and methods described below can be implemented in connection with any suitably configured or configurable IMD. Accordingly, one of skill in the art could readily duplicate, eliminate, or disable the appropriate circuitry in any desired combination to provide a device capable of treating the appropriate chamber(s) or regions of a patient's heart with cardioversion, defibrillation, pacing stimulation, autonomic nerve stimulation, non-myocardial tissue stimulation, other nerve stimulation, etc.

Housing 200 for IMD 100 is often referred to as the "can", "case" or "case electrode", and may be programmably selected to act as the return electrode for all "unipolar" modes. Housing 200 may further be used as a return electrode alone or in combination with one or more of the coil electrodes 126, 132 and 134 for shocking purposes. Housing 200 further includes a connector (not shown) having a plurality of terminals 201, 202, 204, 206, 208, 212, 214, 216, 218, 221 (shown schematically and, for convenience, the names of the electrodes to which they are connected are shown next to the terminals).

To achieve right atrial sensing and/or pacing, the connector includes at least a right atrial tip terminal (A_{R} TIP) 201 adapted for connection to the atrial tip electrode 120. A right atrial ring terminal (A_{R} RING) 202 is also shown, which is adapted for connection to the atrial ring electrode 121. To achieve left chamber sensing, pacing and/or shocking, the connector includes at least a left ventricular tip terminal (V_{L} TIP) 204, a left atrial ring terminal (A_{L} RING) 206, and a left atrial shocking terminal (A_{L} COIL) 208, which are adapted for connection to the left ventricular tip electrode 122, the left atrial ring electrode 124, and the left atrial coil electrode 126, respectively. Connection to suitable autonomic nerve stimulation electrodes or other tissue stimulation or sensing electrodes is also possible via these and/or other terminals (e.g., via a nerve and/or tissue stimulation and/or sensing terminal S ELEC 221).

To support right chamber sensing, pacing, and/or shocking, the connector further includes a right ventricular tip terminal (V_{R} TIP) 212, a right ventricular ring terminal (V_{R} RING) 214, a right ventricular shocking terminal (RV COIL) 216, and a superior vena cava shocking terminal (SVC COIL) 218, which are adapted for connection to the right ventricular tip electrode 128, right ventricular ring electrode 130, the RV coil electrode 132, and the SVC coil electrode 134, respectively. Connection to suitable autonomic nerve stimulation electrodes or other tissue stimulation or sensing electrodes is also possible via these and/or other terminals (e.g., via a nerve and/or tissue stimulation and/or sensing terminal S ELEC 221).

At the core of the IMD 100 is a programmable microcontroller 220 that controls the various modes of stimulation therapy. As is well known in the art, microcontroller 220 typically includes a microprocessor, or equivalent control circuitry, designed specifically for controlling the delivery of stimulation therapy, and may further include RAM or ROM memory, logic and timing circuitry, state machine circuitry, and I/O circuitry. Typically, microcontroller 220 includes the ability to process or monitor input signals (data or information) as controlled by a program code stored in a designated block of memory. The type of microcontroller is not critical to the described implementations. Rather, any suitable microcontroller(s) 220 may be used that carries out the functions described herein. The use of microprocessor-based control circuits for performing timing and data analysis functions are well known in the art.

Fig. 2 also shows an atrial pulse generator 222 and a ventricular pulse generator 224 that generate pacing stimulation pulses for delivery by the right atrial lead 104, the coronary sinus lead 106, and/or the right ventricular lead 108 via an electrode configuration switch 226. It is understood that in order to provide stimulation therapy in each of the four chambers of the heart the atrial and ventricular pulse generators, 222 and 224, may include dedicated, independent pulse generators, multiplexed pulse generators, or shared pulse generators. The pulse generators 222 and 224 are controlled by the microcontroller 220 via appropriate control signals 228 and 230, respectively, to trigger or inhibit the stimulation pulses.

Microcontroller 220 further includes a plurality of modules 232 that, when executed, perform various functions of the IMD. For instance, the modules can perform arrhythmia detection, timing control, and/or morphology detection, among other functionalities.

The illustrated example specifically designates a timing control module 234, an arrhythmia detection module 236, a capture detection module 238, and a thoracic impedance/physiology correlation module 240.

Timing control module 234 controls the timing of the stimulation pulses (e.g., pacing rate, atrio-ventricular (AV) delay, atrial interconduction (A-A) delay, or ventricular interconduction (VV) delay, etc.) as well as to keep track of the timing of refractory periods, blanking intervals, noise detection windows, evoked response windows, alert intervals, marker channel timing, etc., which is well known in the art.

The arrhythmia detection module 236 and the capture detection module 238 can be utilized by the IMD 100 for detecting patient conditions and determining desirable times to administer various therapies such as pacing, defibrillation and/or in vivo dispensing of pharmaceuticals. The thoracic impedance/physiology correlation module 240 provides a mechanism for correlating measured thoracic impedance values with the patient's concurrent posture. Examples of mechanisms for obtaining patient postures and/or measuring thoracic impedance values are described in more detail below.

In some configurations, the thoracic impedance/physiology correlating module 240 can further process the correlated thoracic impedance values and/or serve to diagnose patient conditions and/or effect patient treatment based upon the correlated thoracic impedance values. In one such scenario, the thoracic impedance/physiology correlating module can generate individualized patient-based thresholds for use in diagnosing patient conditions. The individualized patient-based threshold offers a patient specific tool for interpreting thoracic impedance values gathered from the patient. Such capabilities are described in more detail below by way of example. While a functionality of the thoracic impedance/physiology correlation module 240 is described herein in reference to specific components any component and/or process carried out on IMD 100 which senses patient posture and thoracic impedance can potentially benefit from correlation thereof. The aforementioned modules may be implemented in hardware as part of the microcontroller 220, or as software/firmware instructions programmed into the device and executed on the microcontroller 220 during certain modes of operation.

The electronic configuration switch 226 includes a plurality of switches for connecting the desired electrodes to the appropriate I/O circuits, thereby providing complete electrode programmability. Accordingly, switch 226, in response to a control signal 242 from the microcontroller 220, determines the polarity of the stimulation pulses (e.g., unipolar, bipolar, combipolar, etc.) by selectively closing the appropriate combination of switches (not shown) as is known in the art.

Atrial sensing circuits 244 and ventricular sensing circuits 246 may also be selectively coupled to the right atrial lead 104, coronary sinus lead 106, and the right ventricular lead 108, through the switch 226 for detecting the presence of cardiac activity in each of the four chambers of the heart. Accordingly, the atrial (ATR. SENSE) and ventricular (VTR. SENSE) sensing circuits, 244 and 246, may include dedicated sense amplifiers, multiplexed amplifiers, or shared amplifiers. Switch 226 determines the "sensing polarity" of the cardiac signal by selectively closing the appropriate switches, as is also known in the art. In this way, the clinician may program the sensing polarity independent of the stimulation polarity. The sensing circuits (e.g., 244 and 246) are optionally capable of obtaining information indicative of tissue capture.

Each sensing circuit 244 and 246 preferably employs one or more low power, precision amplifiers with programmable gain and/or automatic gain control, bandpass filtering, and a threshold detection circuit, as known in the art, to selectively sense the cardiac signal of interest. The automatic gain control enables the IMD 100 to deal effectively with the difficult problem of sensing the low amplitude signal characteristics of atrial or ventricular fibrillation.

The outputs of the atrial and ventricular sensing circuits 244 and 246 are connected to the microcontroller 220, which, in turn, is able to trigger or inhibit the atrial and ventricular pulse generators 222 and 224, respectively, in a demand fashion in response to the absence or presence of cardiac activity in the appropriate chambers of the heart. Furthermore, as described herein, the microcontroller 220 is also capable of analyzing information output from the sensing circuits 244 and 246 and/or the data acquisition system 252 to determine or detect whether capture has occurred and to program a pulse, or pulses, in response to such determinations. The sensing circuits 244 and 246, in turn, receive control signals over signal lines 248 and 250 from the microcontroller 220 for purposes of controlling the gain, threshold, polarization charge removal circuitry (not shown), and the timing of any blocking circuitry (not shown) coupled to the inputs of the sensing circuits, 244 and 246, as is known in the art.

For arrhythmia detection, IMD 100 utilizes the atrial and ventricular sensing circuits, 244 and 246, to sense cardiac signals to determine whether a rhythm is physiologic or pathologic. In reference to arrhythmias, as used herein, "sensing" is reserved for the noting of an electrical signal or obtaining data (information), and "detection" is the processing (analysis) of these sensed signals and noting the presence of an arrhythmia. The timing intervals between sensed events (e.g., P-waves, R-waves, and depolarization signals associated with fibrillation which are sometimes referred to as "F-waves" or "Fib-waves") are then classified by the arrhythmia detector 236 of the microcontroller 220 by comparing them to a predefined rate zone limit (i.e., bradycardia, normal, low rate VT, high rate VT, and fibrillation rate zones) and various other characteristics (e.g., sudden onset, stability, physiologic sensors, and morphology, etc.) in order to determine the type of remedial therapy that is needed (e.g., bradycardia pacing, anti-tachycardia pacing, cardioversion shocks or defibrillation shocks, collectively referred to as "tiered therapy").

Cardiac signals are also applied to inputs of an analog-to-digital (A/D) data acquisition system 252. The data acquisition system 252 is configured to acquire intracardiac electrogram signals, convert the raw analog data into a digital signal, and store the digital signals for later processing and/or telemetric transmission to an external device 254. The data acquisition system 252 is coupled to the right atrial lead 104, the coronary sinus lead 106, the right ventricular lead 108 and/or the nerve or other tissue stimulation lead 110 through the switch 226 to sample cardiac signals across any pair of desired electrodes.

The microcontroller 220 is further coupled to a memory 260 by a suitable data/address bus 262, wherein the programmable operating parameters used by the microcontroller 220 are stored and modified, as required, in order to customize the operation of the IMD 100 to suit the needs of a particular patient. Such operating parameters define, for example, pacing pulse amplitude, pulse duration, electrode polarity, rate, sensitivity, automatic features, arrhythmia detection criteria, and the amplitude, waveshape, number of pulses, and vector of each shocking pulse to be delivered to the patient's heart 102 within each respective tier of therapy.

Still other examples of parameters that can be stored in memory can include various characterizations of one or more patient conditions, prior to implantation of the IMD, during implantation of the IMD, and/or after implantation of the IMD. In one such example, the memory can be utilized to store a characterization of the patient's cardiac health prior to implantation. The characterization may be based on a classification system, such as the New York Heart Association (NYHA) classes and/or actual parameter values. In another example, parameter values, such as thoracic impedance values, from a first period after implantation can be stored to provide a baseline condition to which sampled values from a second period can be compared to establish if the patient's cardiac condition is improving, steady, or worsening.

Advantageously, the operating parameters of the IMD 100 may be non-invasively programmed into the memory 260 through a telemetry circuit 264 in telemetric communication via communication link 266 with the external device 254, such as a programmer, transtelephonic transceiver, or a diagnostic system analyzer. The microcontroller 220 activates the telemetry circuit 264 with a control signal 268. The telemetry circuit 264 advantageously allows intracardiac electrograms and status information relating to the operation of the device 100 (as contained in the microcontroller 220 or memory 260) to be sent to the external device 254 through an established communication link 266.

The IMD 100 can further include a physiologic sensor(s) 270 to detect one or more of patient activity, patient posture, and respirations, among others. Microcontroller 220 can utilize data received from the physiologic sensor(s) 270 to adjust the various pacing parameters (such as rate, AV Delay, VV Delay, etc.) at which the atrial and ventricular pulse generators, 222 and 224, generate stimulation pulses. Microcontroller 220 further can utilize data received from the physiologic sensor(s) 270 to identify patient postures that can be correlated with measured thoracic impedance values.

While shown as being included within the IMD 100, it is to be understood that the physiologic sensor 270 may also be external to the IMD 100, yet still be implanted within or carried by the patient. Examples of physiologic sensors that may be implemented in IMD 100 include known sensors that, for example, sense pressure, respiration rate, pH of blood, cardiac output, preload, afterload, contractility, oxygen levels, and so forth. Another sensor that may be used is one that detects activity variance, where an activity sensor is monitored to detect the low variance in the measurement corresponding to the sleep state and/or maintenance of a specific posture.

The physiological sensors 270 optionally include sensors for detecting movement and minute ventilation in the patient. The physiological sensors 270 may include a position sensor and/or a minute ventilation (MV) sensor to sense minute ventilation, which is defined as the total volume of air that moves in and out of a patient's lungs in a minute. Signals generated by the position sensor and MV sensor are passed to the microcontroller 220 for analysis in determining whether to adjust the pacing rate, etc. The microcontroller 220 monitors the signals for indications of the patient's posture and activity status, such as whether the patient is climbing upstairs or descending downstairs or whether the patient is sitting up after supine down.

The IMD 100 optionally includes circuitry capable of sensing heart sounds and/or vibration associated with events that produce heart sounds. Such circuitry may include an accelerometer as conventionally used for patient position and/or activity determinations. Accelerometers typically include two or three sensors aligned along orthogonal axes. For example, a commercially available micro-electromechanical system (MEMS) marketed as the ADXL202 by Analog Devices, Inc. (Norwood, Massachusetts) has a mass of about 5 grams and a 14 lead CERPAK (approx. 10 mm by 10 mm by 5 mm or a volume of approx. 500 mm³). The ADXL202 MEMS is a dual-axis accelerometer on a single monolithic integrated circuit and includes polysilicon springs that provide a resistance against acceleration forces. The term MEMS has been defined generally as a system or device having micro-circuitry on a tiny silicon chip into which some mechanical device such as a mirror or a sensor has been manufactured. The aforementioned ADXL202 MEMS includes micro-circuitry and a mechanical oscillator.

Another commercially available MEMS accelerometer is the ADXL330 by Analog Devices, Inc., which is a small, thin, low power, complete three axis accelerometer with signal conditioned voltage outputs, all on a single monolithic IC. The ADXL330 product measures acceleration with a minimum full-scale range of ±3 g. It can measure the static acceleration of gravity in tilt-sensing applications, as well as dynamic acceleration resulting from motion, shock, or vibration. Bandwidths can be selected to suit the application, with a range of 0.5 Hz to 1,600 Hz for X and Y axes, and a range of 0.5 Hz to 550 Hz for the Z axis. Various heart sounds include frequency components lying in these ranges. The ADXL330 is available in a small, low-profile, 4 mm × 4 mm × 1.45 mm, 16-lead, plastic lead frame chip scale package (LFCSP_LQ).

While an accelerometer may be included in the case of an IMD in the form of an implantable pulse generator device, alternatively, an accelerometer communicates with such a device via a lead or through electrical signals conducted by body tissue and/or fluid. In the latter instance, the accelerometer may be positioned to advantageously sense vibrations associated with cardiac events. For example, an epicardial accelerometer may have improved signal to noise for cardiac events compared to an accelerometer housed in a case of an implanted pulse generator device.

IMD 100 may also include, or be in communication with, an implanted drug pump 274 or other drug delivery mechanism to effect patient therapy. The drug pump can be activated in various scenarios, such as when a heart failure condition is detected by thoracic impedance/physiology correlation module 240.

The IMD 100 additionally includes a battery 276 that provides operating power to all of the circuits shown in Fig. 2. For the IMD 100, which employs shocking therapy, the battery 276 is capable of operating at low current drains for long periods of time (e.g., preferably less than 10 µA), and is capable of providing high-current pulses (for capacitor charging) when the patient requires a shock pulse (e.g., preferably, in excess of 2 A, at voltages above 200 V, for periods of 10 seconds or more). The battery 276 also desirably has a predictable discharge characteristic so that elective replacement time can be detected.

The IMD 100 can further include magnet detection circuitry (not shown), coupled to the microcontroller 220, to detect when a magnet is placed over the IMD 100. A magnet may be used by a clinician to perform various test functions of the IMD 100 and/or to signal the microcontroller 220 that the external programmer 254 is in place to receive or transmit data to the microcontroller 220 through the telemetry circuits 264. Trigger IEGM storage also can be achieved by magnet.

The IMD 100 further includes an impedance measuring circuit 278 that is enabled by the microcontroller 220 via a control signal 280. The known uses for an impedance measuring circuit 278 include, but are not limited to, lead impedance surveillance during the acute and chronic phases for proper lead positioning or dislodgement; detecting operable electrodes and automatically switching to an operable pair if dislodgement occurs; measuring respiration or minute ventilation; measuring thoracic impedance, such as for determining shock thresholds, (HF indications - pulmonary edema and other factors); detecting when the device has been implanted; measuring stroke volume; and detecting the opening of heart valves, etc. The impedance measuring circuit 278 is advantageously coupled to the switch 226 so that any desired electrode may be used.

In the case where the IMD 100 is intended to operate as an implantable cardioverter/defibrillator (ICD) device, it detects the occurrence of an arrhythmia, and automatically applies an appropriate therapy to the heart aimed at terminating the detected arrhythmia. To this end, the microcontroller 220 further controls a shocking circuit 282 by way of a control signal 284. The shocking circuit 282 generates shocking pulses in a range of joules, for example, conventionally up to about 40 J, as controlled by the microcontroller 220. Such shocking pulses are applied to the patient's heart 102 through at least two shocking electrodes, and as shown in this embodiment, selected from the left atrial coil electrode 126, the RV coil electrode 132, and/or the SVC coil electrode 134. As noted above, the housing 200 may act as an active electrode in combination with the RV electrode 132, or as part of a split electrical vector using the SVC coil electrode 134 or the left atrial coil electrode 126 (i.e., using the RV electrode as a common electrode).

Cardioversion level shocks are generally considered to be of low to moderate energy level (so as to minimize battery drain and the more rapid delivery of the shock if the lower energy levels are effective in restoring a normal rhythm), and/or synchronized with an R-wave and/or pertaining to the treatment of tachycardia. Defibrillation shocks are generally of moderate to high energy level (i.e., corresponding to thresholds in the range of approximately 5 J to approximately 40 J), delivered asynchronously (since R-waves may be too disorganized), and pertaining exclusively to the treatment of fibrillation. Accordingly, the microcontroller 220 is capable of controlling the synchronous or asynchronous delivery of the shocking pulses.

In low-energy cardioversion, an IMD typically delivers a cardioversion stimulus (e.g., .1 - 5 J, etc.) synchronously with a QRS complex; thus, avoiding the vulnerable period of the T wave and avoiding an increased risk of initiation of VF. In general, if antitachycardia pacing or cardioversion fails to terminate a tachycardia, then, for example, after a programmed time interval or if the tachycardia accelerates, the IMD initiates defibrillation therapy.

While an IMD may reserve defibrillation as a latter tier therapy, it may use defibrillation as a first-tier therapy for VF. In general, an IMD does not synchronize defibrillation therapy with any given portion of a ECG. Again, defibrillation therapy typically involves high-energy shocks (e.g., 5 J to 40 J), which can include monophasic or unidirectional and/or biphasic or bidirectional shock waveforms. Defibrillation may also include delivery of pulses over two current pathways.

### Exemplary Correlation Techniques

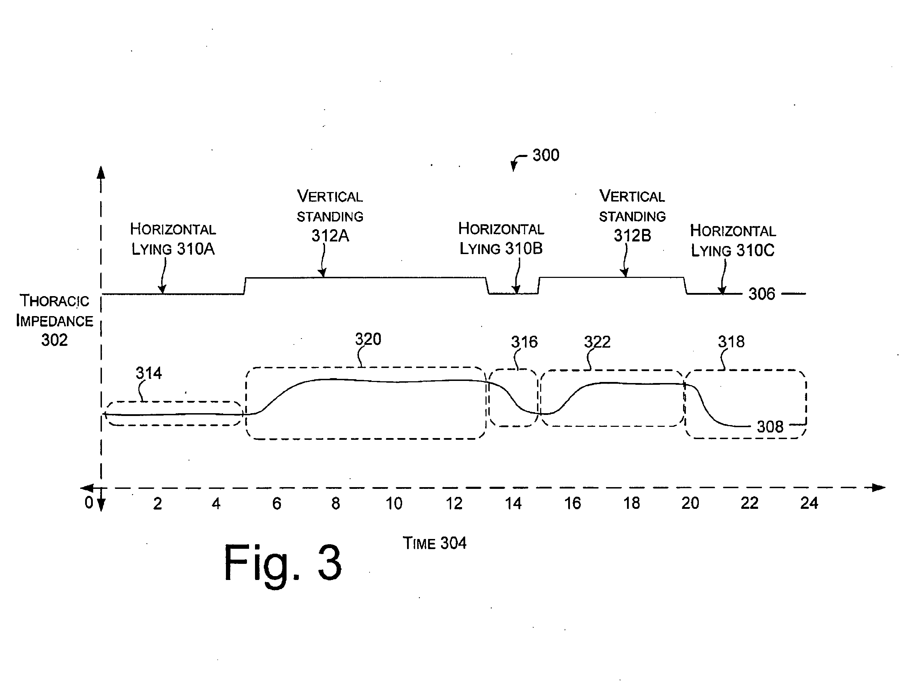

Figs. 3-4 illustrate examples for correlating measured thoracic impedance values with physiological status to increase a prognostic value of the thoracic impedance values.

Figs. 3-4 represent a plot 300 of thoracic impedance values from a patient over time. Thoracic impedance 302 is represented along the vertical axis while time 304 is represented along the horizontal axis with midnight arbitrarily corresponding to time zero and the time extending for an arbitrary sample period of 24 hours. Assume for purposes of explanation that line 306 represents the patient's posture and line 308 represents a compilation of sampled thoracic impedance values. Line 306 represents a patient in a horizontal supine posture at 310A from time zero for approximately five hours until the patient switches to a vertical upright posture 312A. The patient maintains the vertical upright posture 312A until about thirteen hours at which point the patient returns to a horizontal supine posture at 310B. At approximately fifteen hours the patient switches from the horizontal supine posture 310B to a vertical upright posture 312B which is maintained until the patient again lies down in horizontal supine posture 310C at about 20 hours. Assume for purposes of explanation that line 306 is representative of a patient's average day where the patient is lying down and sleeping from midnight until rising about 5:00 A.M. (e.g., 5 hours). The patient then stands and/or sits until about 1:00 P.M. (e.g., 13 hours) at which time the patient lies down to take a nap or rest. After napping the patient again stands or sits until going to bed at about 8:00 P.M. (e.g., 20 hours). For ease of explanation, in this example, patient postures are limited to horizontal (supine) and vertical or upright (standing or sitting). The skilled artisan should recognize that other postures can be handled in a similar manner.

The patient's posture, as evidenced along line 306, can be ascertained utilizing various techniques. Several suitable examples of sensors which can be utilized for determining patient posture are described above in relation to Fig. 2. Any one or a combination of, these sensors can be utilized to ascertain the patient's posture. Alternatively or additionally to utilizing sensors to determine patient posture, plot 300 illustrates that, at least for some patients, such as those having a fairly regular schedule, posture can be determined, solely or in part, by the time of day. For instance, a particular patient may repeatedly lie down between 8:00 P.M. and 5:00 A.M. and be upright between 5:00 A.M. and 1:00 P.M., etc.

Line 308 represents a compilation of many sensed thoracic impedance values for the 24 hour duration of plot 300. Rather than taking an average of the thoracic impedance values for the duration of the plotted sample period (e.g., 24 hours), the present implementations correlate the patient's posture with concurrently measured thoracic impedance values. For instance, thoracic impedance values indicated generally at sub-set 314 were sensed or measured when the patient was in the horizontal supine posture indicated at 310A. Similarly, thoracic impedance values indicated at sub-sets 316 and 318 correspond to horizontal supine postures 310B, 310C respectively. Thoracic impedance values indicated at sub-sets 320 and 322 correspond to vertical upright postures 312A, 312B respectively. Thoracic impedance values that are correlated with the patient's posture can have more prognostic value than traditional techniques, such as averaging impedance values over the sample period. For example, an average of the impedance values from sub-sets 314, 316, and 318 sampled when the patient is supine can be indicative of pulmonary edema resulting from heart failure at an earlier stage than can be detected with existing techniques such as simply taking an average value of all samples from the 24 hour period.

To further enhance the prognostic value of the correlated thoracic impedance values, some implementations average the thoracic impedance values of the sample period measured while the patient is supine (e.g., sub-sets 314, 316, and 318) and separately average the thoracic impedance values measured while the patient is upright (e.g., sub-sets 320, 322) during the sample period. Some of these implementations then calculate a difference between the average supine posture thoracic impedance value and the average upright posture thoracic impedance values. The difference between the upright and supine thoracic impedance values can be indicative of pulmonary edema (resulting from heart failure) at an earlier stage than can be detected with existing techniques.

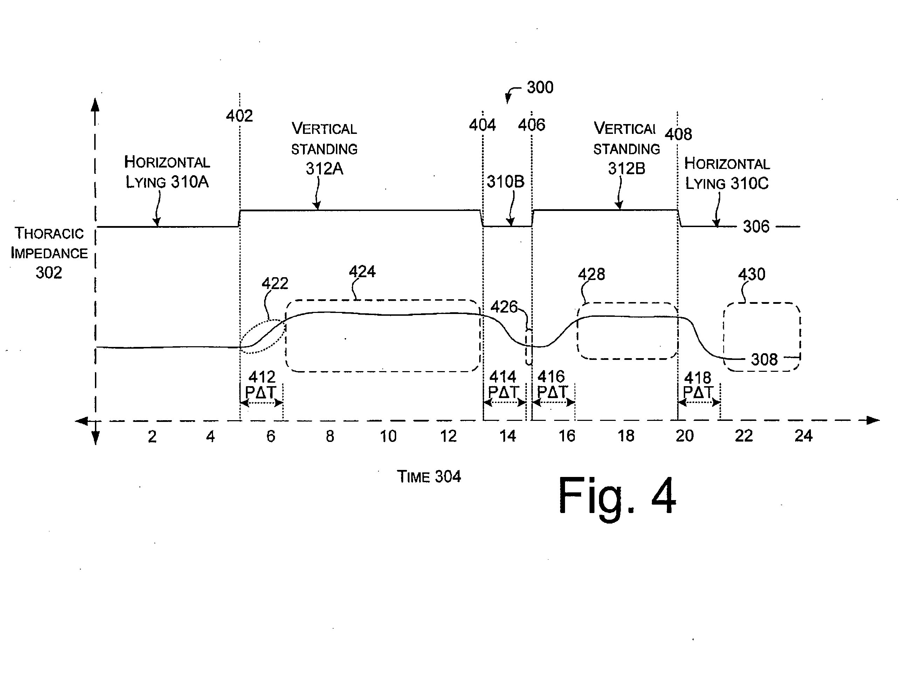

Still other techniques for deriving average thoracic impedance values for supine versus upright postures can be employed. One such technique recognizes that often thoracic impedance values change for a period of time following a posture change whether the heart is functioning properly or not. To this end some exemplary techniques exclude impedance values measured during a predefined period of time after posture changes when attempting to determine average thoracic impedance values for different postures. One such technique can be gleaned from Fig. 4.

Fig. 4 shows an example that restricts which measured thoracic impedance values are utilized to generate average thoracic impedance values for the patient. Patient posture changes are specifically designated with vertical lines at 402, 404, 406 and 408. For instance, patient posture change 402 represents a change from horizontal supine 310A to vertical upright 312A. In this instance, a predefined period (PΔT) is specified after each posture change. In this particular implementation, the predefined periods are approximately two hours. Other shorter and/or longer predefined periods can be employed in various implementations. One example of a predefined period may utilize an average 'recovery time' of a normally functioning heart as the predefined period.

In this case, predefined periods 412, 414, 416, and 418 follow patient posture changes 402, 404, 406, and 408, respectively. Measured thoracic impedance values during the predefined periods are not included for purposes of determining an average thoracic impedance for the new posture. For instance, in relation to posture change 402, a sub-set of thoracic impedance values indicated at 422 and falling within predefined period 412 are not utilized to determine an average vertical upright thoracic impedance value. Instead, a second sub-set of measured values 424 occurring after the predefined period is utilized to calculate the average value. Similarly, sub-set 426 is utilized for horizontal supine posture 310B, sub-set 428 is utilized for vertical upright posture 312B, and sub-set 430 is utilized for horizontal supine posture 310C. Various averaging techniques can be employed to the thoracic impedance values. For instance, one technique pools the values from the horizontal supine postures (e.g. the measured thoracic impedance values from sub-sets 426 and 430) and determines an average from the pooled values. (While values from 0 to 5 hours are not specifically discussed in this example, these sampled values can also be utilized to calculate the average). Similarly, the values from the vertical upright posture are pooled (e.g., the measured thoracic impedance values from sets 424 and 428) and an average determined from the pooled values. A difference of the average vertical upright value and the average horizontal supine value can then be determined and utilized as described above and below.

The skilled artisan should recognize that the above described techniques offer still other advantages. For example, IMDs may measure upwards of thousands of thoracic impedance values per day. Given that IMDs possess limited energy and processing capacities, the capability of recognizing the measured thoracic impedance values which are of relatively higher diagnostic value can be especially valuable in that a reduced number of measured thoracic impedance values can be processed from a given sample period. For instance in the above example of Fig. 4, avoiding further processing of thoracic impedance values measured in the predefined periods after posture changes saves energy and processing resources. Alternatively or additionally, correlating the measured thoracic impedance values with patient posture may reduce the number of those thoracic impedance values which have to be processed to determine accurate average values for the sample period. For example, within an identified sub-set, of the sample period where the patient maintains a specific posture, processing relatively few of the measured values, such as 1 in a hundred or 1 in a thousand may provide a relatively accurate average thoracic impedance. In summary, correlating the measured thoracic impedance values and patient posture allows earlier detection of patient conditions and/or allows the IMD to select which thoracic impedance values are most useful for determining various patient conditions and hence warrant further processing.

While Figs. 3-4 are described in relation to patient posture, the thoracic impedance values can alternatively or additionally be correlated to other aspects of the patient's physiological status. For instance, thoracic impedance values can be correlated to the patient's activity level. Some implementations may concurrently track multiple aspects. So for instance, an implementation might track both activity level and posture. The two aspects could be combined so that separate average thoracic impedance values could be tracked for various combinations, such as upright and active, upright and resting, supine and active and supine and resting. A near limitless number of physiological aspects could be correlated to measured thoracic impedance values. For example, in a particular scenario it may be advantageous to correlate measured thoracic impedance values with blood sodium ion concentrations. The skilled artisan should recognize variations consistent with these concepts.

### Exemplary Tracking Techniques

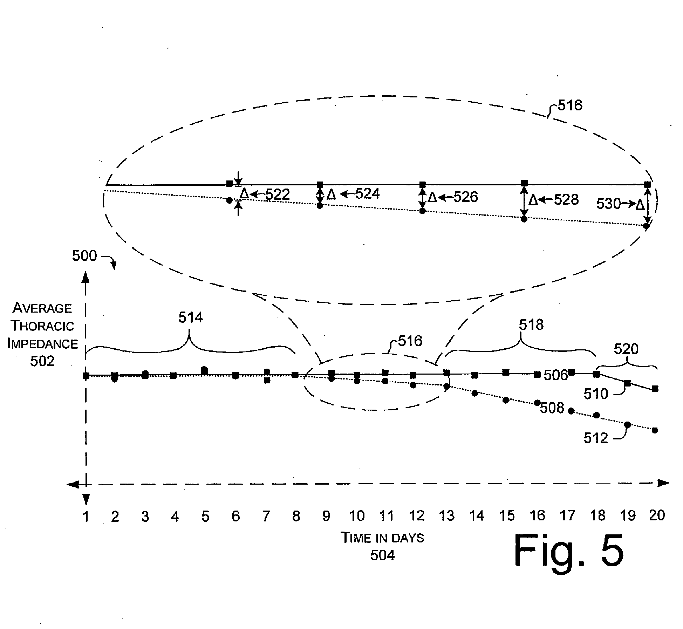

Fig. 5 represents a hypothetical plot 500 of daily average thoracic impedance values from a patient over time. Average thoracic impedance 502 is represented along the vertical axis. Time 504 is represented along the horizontal axis as 20 individual days (e.g., days 1-20). For individual days an average upright thoracic impedance value and an average supine thoracic impedance value are represented on plot 500. Each day's average upright thoracic impedance value is represented as a square 510 (not all of which are designated with specificity). Each day's average supine thoracic impedance value is represented as a circle 512 (not all of which are designated with specificity). Exemplary techniques for determining an average upright thoracic impedance value and a supine thoracic impedance value for individual days are described above the relation to Figs. 3-4.

In this case, for a first period 514, including days 1-8, the average upright thoracic impedance value and the average supine thoracic impedance values are essentially identical and are not readily distinguishable from one another. A second period 516 begins at day 9 and runs through day 13. Second period 516 is shown both in the context of plot 500 and in an enlarged view illustrated above the plot for explanation purposes as should become apparent below. In second period 516 the average upright thoracic impedance values and the average supine thoracic impedance values begin to diverge as the supine values get progressively lower. In this scenario, the decreasing supine values can indicate that the heart is having trouble handling fluid mobilized when the patient lies down. The heart is functioning well enough that the vertical upright values remain relatively constant.

A third period 518 begins at day 14 and extends through day 18. In third period 518 the horizontal thoracic impedance values drop at a faster rate than in the second period. Stated another way, line 508 has a steeper slope in the third period 518 than second period 516. The rapidly decreasing horizontal supine values of the third period indicate that the heart is having even more trouble handling increased fluid loads associated with supine postures. In a fourth period 520 beginning at day 18 and continuing through day 20, both the supine thoracic impedance values and the upright thoracic impedance values are dropping. The fourth period is indicative of a later stage of heart failure where the patient's heart is falling behind when the patient is in either the upright or supine postures.

Tracking daily average supine thoracic impedance values versus daily upright thoracic impedance values is effective for detecting various stages of heart failure, such as the stages corresponding to periods 516-520. Tracking daily supine thoracic impedance values versus daily upright thoracic impedance values is especially useful for detecting early stages of heart failure which might be missed utilizing other thoracic impedance tracking techniques. Detection of early stage heart failure allows more treatment options and offers an increased quality of life for the patient. In the illustrated example, the present techniques detect small differences (Δs) in the average thoracic impedance values between the upright and supine postures. The differences between average upright and supine thoracic impedance values first appear at day nine in a difference referenced by designator 522. Days 10-13 show progressively larger differences between the average upright and supine thoracic impedance values as indicated at 524, 526, 528 and 530, respectively. Other techniques, such as simply tracking a daily thoracic impedance value, may not provide meaningful indication of heart failure at these early stages. Further, tracking both daily average upright and average supine thoracic impedance values provides additional useful information. For instance, consider third period 518 and fourth period 520. In third period 518, as evidenced by the decreasing average supine thoracic impedance values, the patient's heart is unable to deal with the mobilized fluid associated with supine postures. However, as indicated by the relatively steady average upright thoracic impedance values, the heart is still able to perform fairly normally when the patient is upright. Conversely, in fourth period 520, both the average daily supine thoracic impedance values and the average daily upright thoracic impedance values are decreasing. The fourth period 520 is consistent with a scenario where the patient's heart is failing generally and posture change is no longer a potential treatment option. A daily running average impedance value would not readily distinguish between the two scenarios representative of third and fourth periods 518, 520.

### Operation

### First example - Exemplary individualization Techniques

Various techniques for correlating measured thoracic impedance values with patient physiology to enhance prognostic value of the measured values are described above in relation to Figs. 3-4. Fig. 5 offers techniques for tracking the correlated thoracic impedance measurements over time to further enhance the prognostic value. Still other techniques are offered in this section for customizing or individualizing thoracic impedance values to a particular patient to more accurately diagnose a condition of the patient.

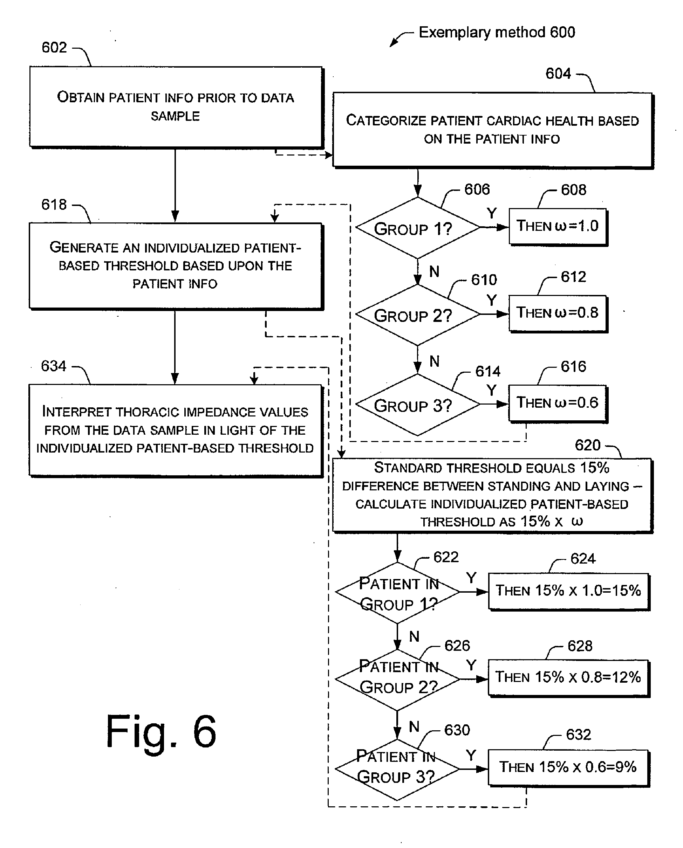

Fig. 6 shows an exemplary method or technique 600 for individualizing thoracic impedance values to a particular patient to more accurately diagnose a condition of the patient. This technique 600 may be implemented in connection with any suitably configured implantable medical devices (IMDs) and/or systems such as those described above. Technique 600 includes blocks 602-634. An exemplary basic method is described as a set of blocks 602, 618, and 634 which appear on the left side of the physical page upon which Fig. 6 appears. An example of a technique for implementing block 602 appears on the right side of the page as blocks 604-616. Similarly, an example for implementing block 618 is described on the right side of the page as blocks 620-632. The order in which the method is described is not intended to be construed as a limitation, and any number of the described blocks can be combined in any order to implement the technique, or an alternate technique. Furthermore, the techniques can be implemented in any suitable hardware, software, firmware, or combination thereof.

At block 602, patient information is obtained prior to a data sample. The patient information can relate to, for instance, cardiac function and can be obtained in various scenarios. For example, the patient information may be obtained from the patient before and/or proximate to implantation of an implantable medical device or by examination of the patient during or after implantation of the IMD. The patient information may relate to various physiological parameters. For instance, physiological parameters, such as echo parameters and pressures may be obtained during the implantation procedure utilizing known techniques.

In one case, at block 604, the patient's cardiac health is categorized based on the patient information. Many suitable categorization themes or scaling factors may be employed. For instance, one widely known categorization theme is defined by the New York Heart Association (NYHA) classes. Another categorization involves left atrial pressure such as may be determined during the implantation procedure. This particular categorization is divided into three patient classes indicated in Fig. 6 as group 1, group 2, and group 3.

At block 606, the technique inquires whether the patient is in group 1. Group 1 is defined as a left atrial pressure of less than x where x is a typical value for a NYHA class II patient. If the patient is a group 1 patient (e.g., a yes at block 606 then a variable ω is assigned a value of 1.0 at block 608. If the patient is not a group 1 patient (e.g., no at block 606) then the technique proceeds to block 610. As should become apparent below, variable ω functions as a weighting coefficient to individualize a categorization of the patient.

At block 610, the technique inquires whether the patient is in group 2. In this example group 2 patients have a left atrial pressure greater than x and less than a second variable y where y is a typical value for patients with fluid retention or an NYHA class III - IV. If the patient is a group 2 patient (e.g., a yes at block 610, then the variable ω is assigned a value of 0.8 at block 612. If the patient is not a group 2 patient (e.g., no at block 610 then the technique proceeds to block 614.

At block 614 the technique inquires whether the patient is a group 3 patient. If the patient is a group 3 patient, then the technique proceeds to block 616 where the variable ω is assigned a value of 0.6. Having categorized the patient and assigned a value to the variable ω, the process returns to the left side of the figure at block 618.

An individualized patient-based threshold based upon the patient information is generated at block 618. In some implementations, the individualized patient based-threshold can be based upon a standard threshold for interpreting patient thoracic impedance values. The standard threshold is individualized to the patient via the patient information to generate the individualized patient-based threshold. The individualized patient-based threshold offers a patient specific tool for interpreting the patient's thoracic impedance values, such as those sensed by an IMD.

Consider blocks 620-632 as offering one example of how a standard threshold can be individualized with the patient information to generate an individualized patient-based threshold.

Block 620 recites a standard threshold for detecting heart failure. For purposes of explanation, assume that in this example the standard threshold equals a 15% difference between average upright and average supine thoracic impedance values. Stated another way the standard threshold defines a 15% or greater difference between the average upright and average supine thoracic impedance values as an indication of heart failure. Conversely, the standard threshold defines a difference between the average upright and average supine thoracic impedance values of less than 15% as indicating more normal heart function. Block 620 calculates an individualized patient base threshold as the standard threshold of 15% multiplied by the variable ω. (The value of the variable w is calculated above in relation to blocks 604-616). In order to calculate the individualized patient-based threshold from the standard threshold the technique proceeds to block 622.

The technique queries whether the patient is in group 1 at block 622. If the patient is a group 1 patient (e.g., a yes at block 622) then the technique proceeds to block 624. If the patient is not a group 1 patient (e.g., no at block 622) then the technique proceeds to block 626.

At block 624, the technique calculates the individual patient-based threshold as 15% times the variable ω. In this instance as determined at 606-608, the variable ω has a value of 1.0. As a result the individualize patient-based threshold equals 15% as the product of 15% multiplied by 1.0.

Returning to the negative instance at block 622, the technique queries whether the patient is in group 2 at block 626. If the patient is a group 2 patient (e.g., a yes at block 626) then the technique proceeds to block 628. If the patient is not a group 2 patient (e.g., no at block 626) then the technique proceeds to block 630.

At block 628, the technique calculates the individual patient-based threshold as 15% times the variable w. In this instance as determined at 610-612, the variable ω has a value of 0.8. As a result the individualize patient-based threshold equals 12% as the product of 15% multiplied by 0.8.

Returning to the negative instance at block 626, the technique queries whether the patient is in group 3 at block 630. If the patient is a group 3 patient (e.g., a yes at block 630) then the technique proceeds to block 632.

At block 632, the technique calculates the individual patient-based threshold as 15% times the variable w. In this instance as determined at blocks 614-616, the variable ω has a value of 0.6. As a result, the individualize patient-based threshold equals 9% as the product of 15% multiplied by 0.6. Blocks 620-632 provide an example for calculating individualized patient-based thresholds from standard thresholds. Possessing an individualized patient-based threshold, the technique returns to block 634 on the left side of the printed page.

At block 634, the technique interprets thoracic impedance values from the data sample in light of the individualized patient-based threshold. For example, differences between average supine thoracic impedance values and average upright thoracic impedance values plotted in Fig. 5 can be interpreted in light of the individualized patient-based threshold to detect a heart failure condition. Interpreting the thoracic impedance values with the individualized patient-based threshold allows more accurate detection of heart failure than can be obtained with standard thresholds.

The above example applies the individualized patient-based threshold to differences between average supine thoracic impedance values and average upright thoracic impedance values. Other examples can apply the individualized patient-based threshold to interpret other facets of the thoracic impedance values. In one case, the individualized patient-based threshold is utilized to interpret two facets of the thoracic impedance values. In one such case, assume that the patient is assigned a group 2 categorization and so the individualized patient-based threshold is assigned a value of 12% as described above at block 628. Daily thoracic impedance values are interpreted in light of the individualized patient-based threshold. If on an individual day, the difference between the average supine thoracic impedance values and average upright thoracic impedance values equals or exceeds the 12% threshold then a first action may be taken. For instance, the first action may be sounding an alarm, such as on an external IMD device manager, or changing a patient therapy delivered by the IMD. A daily average thoracic impedance may also be interpreted in light of the individualized patient-based threshold. For instance, if on the individual day, an overall average thoracic impedance value is less than a running daily overall average by 12% or more, then a second action may be taken such as sounding a second alarm. Such a scenario may be encountered with rapid deterioration of the patient's cardiac health.

While the method of Fig. 6 is described in a beginning-to-end sequential manner for ease of explanation, such need not be the case. For instance, consider a situation consistent with block 602, where a first set of information obtained during implantation of an IMD indicates that the patient is a group 1 patient according to blocks 604-616. A patient-based threshold is then generated at block 618. Assume that a second set of data is collected from the patient during some subsequent period, such as a weeklong period that the patient is recovering in the hospital from the implantation procedure. The second set of data can also be utilized to categorize the patient's cardiac health (such as by the process of blocks 604-616). Having two categorizations offers several options for assessing and/or treating the patient. For instance, if the second categorization (i.e., from the second data set) is the same as the first categorization (i.e., group 1), it serves to reaffirm the first categorization. In contrast, if the second categorization is different from the first categorization (say group 3 versus group 1), a change in the patient's condition may have occurred or the first categorization may have been inaccurate. Toward this end, various actions can be taken. In one case the discrepancy between the two categorizations can be brought to the attention of a treating clinician. In another case the existence of the two differing categorizations can be utilized by the IMD. For instance, the second (i.e., more recent categorization may be utilized to generate an individualized patient-based threshold (such as via the method described by blocks 620-632) that augments or supplants the previous individualized patient-based threshold. In another variation, the clinician may update the patient categorization utilized by the IMD such as via an external programmer. In still another instance, the IMD can also obtain patient data from a third sample period and compare a patient categorization obtained therefrom to the first two patient categorizations. This example illustrates that the exemplary method described in relation to blocks 602-632 or similar methods can be utilized in any useful manner which employs the entire method, portions of the method or repeated applications of the method or its parts. The above examples serve to illustrate that patient treatment can be improved by allowing measured thoracic impedance values to be interpreted according to a threshold that is tailored to the patient.

### Second Example

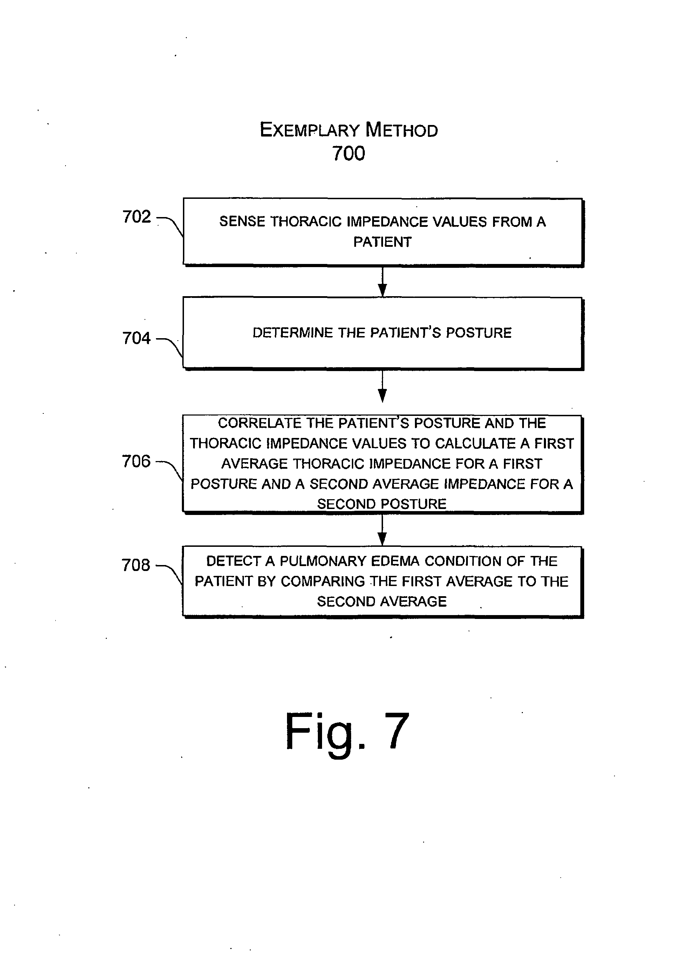

Fig. 7 shows an exemplary method 700 for detecting a patient's pulmonary edema condition by comparing the patient's posture correlated thoracic impedance values. This method 700 may be implemented in connection with any suitably configured implantable medical devices (IMDs) and/or systems such as those described above. Method 700 is described as a series of method blocks 702-708. The order in which the method is described is not intended to be construed as a limitation, and any number of the described method blocks can be combined in any order to implement the method, or an alternate method. Furthermore, the method can be implemented in any suitable hardware, software, firmware, or combination thereof.

Thoracic impedance values are sensed from a patient at block 702. The thoracic impedance values can be sensed in any known manner. For instance, IMDs configured to sense and/or pace cardiac tissue can often be utilized to sense thoracic tissue including the lungs and/or the pulmonary vasculature. An example of such an IMD is described above in relation to Figures 1 and 2.

The patient's posture is determined at block 704. The patient's posture can be determined utilizing any suitable technique or combination of techniques. For instance, various posture sensors and accelerometers can be employed to determine the patient posture. Alternatively or additionally some techniques can utilize time as it relates to the patient's schedule to determine patient posture. For instance, the patient may tend to lie down during certain times of the day and be upright during other times of the day. Thus the patient's posture can be determined at least in part based upon the time of day. Some techniques which utilize time of day (e.g. schedule) to determine patient posture also confirm the schedule based posture with input from one or more sensors. So for instance, in a case where a patient's schedule indicates that he/she should be sleeping, but one or more sensors indicate otherwise, data may be discarded or otherwise dealt with appropriately.

The patient's posture and the thoracic impedance values are correlated to calculate a first average thoracic impedance for a first posture and a second average impedance for a second posture at block 706. Some methods exclude thoracic impedance values that are measured or obtained for a pre-defined period after a posture change is detected. Thoracic impedance values measured after the patient has maintained a particular posture for a period of time can more accurately reflect the patient condition than those taken soon after a posture change. For example, thoracic impedance values measured soon after a posture change can be influenced by the patient's previous posture and/or other variables.

At block 708, the method detects a pulmonary edema condition of the patient by comparing the first average to the second average. Comparing the first and second averages, among other advantages, serves to detect early stages of heart failure. For instance, a heart that is barely able to satisfy the patient's requirements when the patient is upright may fall behind when the patient lies down and effectively mobilizes additional fluids that had been held in the extremities. The principle postures described above in relation to Fig. 3-5 involve upright and supine though thoracic impedance values may be correlated to other and/or additional postures. For ease of explanation, Figs. 3-5 provide examples of how the correlated thoracic impedance values can be tracked graphically. Of course, many implementations can accomplish a similar functionality without actually plotting the values. Some implementations compare the differences between the first and second averages in light of an individualized patient-based threshold. The patient-based threshold is customized to the cardiac state of the individual patient. Accordingly, the patient-based threshold can contribute to enhanced patient diagnosis when compared to more standardized techniques and/or thresholds. Various techniques can be implemented in an instance where the difference exceeds the patient-based threshold. For example, various parameters of the IMD may be adjusted to supply responsive patient therapy. In but one example, a pacing rate may be adjusted when the patient-based threshold is exceeded. The skilled artisan should recognize variations consistent with these concepts.

## Claims

1. An implantable medical device (IMD) comprising:
a mechanism (270) operable to monitor patient postures to detect posture changes between a first posture and a second posture different from the first posture;
a mechanism (278) operable to measure patient thoracic impedance values;
a correlating mechanism (240) operable to correlate the first posture with individual thoracic impedance values measured when the first posture is detected, and the second posture with individual thoracic impedance values measured when the second posture is detected;
a mechanism (234) operable to calculate a difference between thoracic impedance measurements associated with the first posture and thoracic impedance measurements associated with the second posture, wherein the difference is indicative of heart failure stage, from the correlated individual postures and individual thoracic impedance values,
wherein said correlating mechanism excludes thoracic impedance values that occur within predefined periods of time after detected posture changes.

2. An IMD as claimed in Claim 1, **characterised in that** the mechanism to detect calculates an average thoracic impedance value associated with a horizontal posture for an individual day of a plurality of days and compares the average for the individual day to averages for other days of the plurality of days.

3. An IMD as claimed in any preceding claim, **characterised in that** the patient condition comprises pulmonary edema.

4. An IMD as claimed in Claim 3, **characterised in that** the mechanism operable to calculate utilizes a scaling factor that takes into account an incidence of pulmonary edema prior to implantation.

5. An IMD as claimed in Claim 4, **characterised in that** the scaling factor comprises a weighting coefficient.

6. An IMD as claimed in Claim 5, **characterised in that** the weighting coefficient is based upon patient information including echo parameters obtained in proximity to implantation of the implantable medical device.

## Patentansprüche

1. Implantierbare medizinische Vorrichtung (IMD), aufweisend:
einen Mechanismus (270), betriebsfähig zum Überwachen von Lagen eines Patienten, um Lageänderungen zwischen einer ersten Lage und einer zweiten Lage, die von der ersten Lage unterschiedlich ist, zu detektieren;
einen Mechanismus (278), betriebsfähig zum Messen thorakaler Impedanzwerte des Patienten;
einen korrelierenden Mechanismus (240), betriebsfähig zum Korrelieren der ersten Lage mit individuellen thorakalen Impedanzwerten, die beim Detektieren der ersten Lage gemessen werden, und der zweiten Lage mit individuellen thorakalen Impedanzwerten, die beim Detektieren der zweiten Lage gemessen werden;
einen Mechanismus (234), betriebsfähig zum Berechnen einer Differenz zwischen thorakalen Impedanzmessungen im Zusammenhang mit der ersten Lage und thorakalen Impedanzmessungen im Zusammenhang mit der zweiten Lage, wobei die Differenz indikativ für ein Stadium einer Herzinsuffizienz ist, aus den korrelierten individuellen Lagen und individuellen thorakalen Impedanzwerten,
wobei der korrelierende Mechanismus thorakale Impedanzwerte ausschließt, die innerhalb vordefinierter Zeiträume nach dem Detektieren von Lageänderungen auftreten.

2. IMD nach Anspruch 1, **dadurch gekennzeichnet, dass** der Mechanismus zum Detektieren einen durchschnittlichen thorakalen Impedanzwert im Zusammenhang mit einer horizontalen Lage für einen individuellen Tag einer Mehrzahl von Tagen berechnet und den Durchschnitt für den individuellen Tag mit Durchschnittswerten für andere Tage der Mehrzahl von Tagen vergleicht.

3. IMD nach einem der vorhergehenden Ansprüche, **dadurch gekennzeichnet, dass** der Patientenzustand Lungenödeme umfasst.

4. IMD nach Anspruch 3, **dadurch gekennzeichnet, dass** der zum Berechnen betriebsfähige Mechanismus einen Skalierungsfaktor nutzt, der eine Inzidenz von Lungenödemen vor der Implantation berücksichtigt.

5. IMD nach Anspruch 4, **dadurch gekennzeichnet, dass** der Skalierungsfaktor einen Gewichtungskoeffizienten umfasst.

6. IMD nach Anspruch 5, **dadurch gekennzeichnet, dass** der Gewichtungskoeffizient auf Patienteninformationen basiert, die in der Umgebung der Implantation der implantierbaren medizinischen Vorrichtung erhaltene Echoparameter enthalten.

## Revendications

1. Dispositif médical implantable (IMD) comprenant :
un mécanisme (270) pouvant fonctionner pour surveiller des postures d'un patient afin de détecter des changements de posture entre une première posture et une seconde posture différente de la première posture ;
un mécanisme (278) pouvant fonctionner pour mesurer des valeurs d'impédance thoracique d'un patient ;
un mécanisme de corrélation (240) pouvant fonctionner pour corréler la première posture avec des valeurs d'impédance thoracique individuelles mesurées lorsque la première posture est détectée, et la seconde posture avec des valeurs d'impédance thoracique individuelles mesurées lorsque la seconde posture est détectée ;
un mécanise (234) pouvant fonctionner pour calculer une différence entre des mesures d'impédance thoracique associées à la première posture et des mesures d'impédance thoracique associées à la seconde posture, la différence indiquant un niveau d'insuffisance cardiaque, à partir des postures individuelles corrélées et des valeurs d'impédance thoracique individuelles,
dans lequel ledit mécanisme de corrélation exclut les valeurs d'impédance thoracique qui surviennent sur des périodes prédéfinies après des changements de posture détectés.

2. IMD selon la revendication 1, **caractérisé en ce que** le mécanisme de détection calcule une valeur d'impédance thoracique moyenne associée à une posture horizontale pour un jour individuel d'une pluralité de jours et compare la moyenne pour le jour individuel aux moyennes pour d'autres jours de la pluralité de jours.

3. IMD selon une quelconque des revendications précédentes, **caractérisé en ce que** l'état du patient comprend un oedème pulmonaire.

4. IMD selon la revendication 3, **caractérisé en ce que** le mécanisme pouvant fonctionner pour le calcul utilise un facteur d'échelle qui prend en compte une incidence d'oedème pulmonaire avant l'implantation.

5. IMD selon la revendication 4, **caractérisé en ce que** le facteur d'échelle comprend un coefficient de pondération.

6. IMD selon la revendication 5, **caractérisé en ce que** le coefficient de pondération est basé sur des informations du patient incluant des paramètres d'écho obtenus à proximité de l'implantation du dispositif médical implantable.
